# EUROPEAN PATENT SPECIFICATION

(11) **EP 3 398 526 B1**
(45) Date of publication and mention of the grant of the patent: **25.06.2025**
(21) Application number: 18170665.6
(22) Date of filing: 03.05.2018
(51) Int. Cl.: A61B 17/04, A61B 17/80

(54) **BONE FRACTURE PLATES UND SYSTEMS**
FRAKTURPLATTEN UND SYSTEME
PLAQUES DE FRACTURE ET SYSTÈMES

(30) Priority: 05.05.2017 US 201715587473
(43) Date of publication of application: 07.11.2018
(73) Proprietor: Globus Medical, Inc., Audubon, PA 19403 (US)
(72) Inventor: GAULT, James A., Philadelphia, PA 19147 (US); DAVISON, Andrew, Downingtown, PA 19335 (US); BADA, Alex, Phoenixville, PA 19460 (US); SCOLARO, John, Irvine, CA 92604 (US)
(74) Representative: Morabito, Sara

(56) References cited:
- EP-A1- 2 273 943
- EP-A2- 2 227 160
- US-A1- 2006 173 458
- US-A1- 2008 051 786
- US-A1- 2016 030 035
- US-A1- 2017 056 081

## Description

### FIELD

The present disclosure relates to surgical devices, and more particularly, stabilization systems, for example, for trauma applications.

### BACKGROUND

Bone fractures are often repaired by internal fixation of the bone, such as diaphyseal bone, including tibia and fibula bones, using one or more plates. The plate is held against the fractured bone with screws, for example, which engage the bone and heads which provide a compressive force against the plate. The plate and bone are thus forced against each other in a manner that transfers load primarily between a bone contacting surface of the plate and the bone surface to reinforce the fractured bone during healing. This manner of plating generally creates relatively low stress concentration in the bone, as there may be a large contact area between the plate and the diaphyseal bone surface permitting transfer of load to be dispersed. There may be a desire to use locking screws, non-locking screws, or a combination of both that are able to dynamically compress the bone. Of course, the designs of the plates, types of screws, and locking and/or non-locking capabilities may vary based on the location and type of fracture.

US2017/056081, US2016/030035, US2006/173458, US2017/056081 and US2008/051786 describe devices known in the art.

Accordingly, there is a need for plating systems that provide stabilization to the appropriate anatomical area while providing appropriate locking and/or unlocking capability for dynamic compression of the bone.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

According to the invention it is provided a plate according to claim 1. Further advantageous aspects of the invention are set forth in the dependent claims

According to embodiments, a stabilization system may include a plurality of plates configured to fix bone in the treatment of ankle fractures. Anatomic bone plates facilitate the well-known and established treatment methods for bone fractures. An advantage of the exemplary stabilization systems is the availability to use various treatment options. It is often a surgeon's preference whether to use a screw or a suture button system to repair a syndesmosis, and it is advantageous to provide a plate that can accept either.

In one example, the stabilization system comprises a bone plate having an upper surface and a lower surface configured to be in contact with bone. The bone plate has a through-opening extending from the upper surface to the lower surface. The through-opening includes a threaded portion proximate to the lower surface and a non-threaded portion proximate to the upper surface. A fastener is configured to engage the through-opening and to secure the bone plate to the bone. The through-opening is configured to receive one of a locking fastener and a compression fastener.

In another example, the stabilization system comprises a bone plate having an upper surface and a lower surface configured to be in contact with bone. The bone plate has a through-opening extending from the upper surface to the lower surface. The through-opening includes a threaded portion proximate to the lower surface and a non-threaded portion proximate to the upper surface. A locking fastener is configured to be received by the through-opening and configured to be inserted into the bone. The locking fastener has a threaded head portion configured to lock to the bone plate. A compression fastener is configured to be received by the through-opening and configured to be inserted into the bone. The compression fastener has a substantially smooth portion configured to dynamically compress the bone.

In an embodiment, a stabilization system comprises a bone plate having an upper surface and a lower surface configured to be in contact with bone. The bone plate has a through-opening extending from the upper surface to the lower surface. The through-opening is formed by at least three different co-axial bores including a first bore having an internal thread and a first diameter; a second bore having an unthreaded conical side wall and a second diameter, greater than the first diameter; and a third bore having an annular surface surrounding the side wall and a third diameter, greater than the second diameter.

The stabilization system comprises a bone plate having an elongate body extending from a proximal end to a distal end along a longitudinal axis and having an enlarged head portion proximate the distal end, wherein the bone plate is generally symmetric about the longitudinal axis, the bone plate having an elongated slot located along the longitudinal axis having a length greater than its width, the bone plate having a plurality of syndesmotic openings located along the longitudinal axis, wherein one of the syndesmotic openings is located adjacent to the elongated slot, the syndesmotic openings being sized and dimensioned to accept a suture button, a non-locking screw, or a locking screw, and the bone plate having a plurality of openings in the enlarged head portion.

In yet another example, a stabilization system comprises a bone plate having an elongate body extending from a proximal end to a distal end along a longitudinal axis and having an enlarged head portion proximate the distal end, wherein the bone plate is symmetric about the longitudinal axis, the bone plate having first and second stacked openings located along the longitudinal axis and positioned proximate to the proximal end of the bone plate, the first and second stacked openings configured to accept either locking or non-locking fasteners, the bone plate having an elongated slot located along the longitudinal axis having a length greater than its width, wherein the elongated slot is located adjacent to the second stacked opening, the bone plate having a plurality of syndesmotic openings located along the longitudinal axis, wherein one of the syndesmotic openings is located adjacent to the elongated slot, the syndesmotic openings being sized and dimensioned to accept a suture button, a non-locking screw, or a locking screw, and the bone plate having a plurality of openings in the enlarged head portion.

Also described are additional stabilization systems, bone plates, and kits including bone plates, fasteners, and components for installing the same.

It is further described but does not fall within the scope of the invention a stabilization system comprising: a bone plate having an elongate body extending from a proximal end to a distal end along a longitudinal axis and having an enlarged head portion proximate the distal end, wherein the bone plate is symmetric about the longitudinal axis, the bone plate having first and second stacked openings located along the longitudinal axis and positioned proximate to the proximal end of the bone plate, the first and second stacked openings configured to accept either locking or non-locking fasteners, the bone plate having an elongated slot located along the longitudinal axis having a length greater than its width, wherein the elongated slot is located adjacent to the second stacked opening, the bone plate having a plurality of syndesmotic openings located along the longitudinal axis, wherein one of the syndesmotic openings is located adjacent to the elongated slot, the syndesmotic openings being sized and dimensioned to accept a suture button, a non-locking screw, or a locking screw, and the bone plate having a plurality of openings in the enlarged head portion.

In a version, the stabilization system further comprises a first fastener configured to engage the elongated slot to secure the bone plate to the bone.

In another version the stabilization system further comprises a second fastener configured to engage one of the syndesmotic openings, wherein the second fastener is sized and dimensioned to engage the tibia and the fibula.

In another version the plurality of openings in the enlarged head portion comprise conically-threaded locking holes.

In another version one of the plurality of openings in the enlarged head portion is a non-threaded hole configured to receive a non-locking fastener in order to compress the plate to the bone.

In a further version an edge of the bone plate includes a scalloped section.

In a further version the plurality of syndesmotic openings includes first, second, and third syndesmotic openings, wherein the plurality of openings in the enlarged head portion includes a first locking hole positioned proximate to the third syndesmotic opening, and wherein the scalloped section includes a first cutout positioned along the edge between the second and third syndesmotic holes and a second cutout positioned along the edge between the third syndesmotic hole and the first locking hole.

In still another version the enlarged head portion is contoured to mimic the lateral malleolus of the fibula.

In a further version the bone plate is configured to contact the distal fibula.

In another version the bone plate is generally symmetric about the longitudinal axis such that plate can accommodate left or right fibulas.

### BRIEF DESCRIPTION OF THE DRAWINGS

Other aspects, features, and advantages of the present invention will become more fully apparent from the following detailed description, the appended claims, and the accompanying drawings in which like reference numerals identify similar or identical elements.
FIG. 1A is a perspective view of a lateral distal fibula plate according to a first exemplary embodiment;
FIG. 1B is an enlarged view of a distal end of the lateral distal fibula plate shown in FIG. 1A affixed to a bone;
FIG. 2A-2C is an alternative lateral distal fibula plate according to another embodiment;
FIG. 3 is a sectional view of a first (shaft) through-opening extending through the lateral distal fibula plate shown in FIG. 1A with a non-locking fastener inserted in the through-opening;
FIG. 3A is a sectional view of the first through-opening extending through the lateral distal fibula plate shown in FIG. 1A with a locking fastener inserted in the through-opening;
FIG. 4 is a sectional view of a second (syndesmotic) hole extending through the lateral distal fibula plate shown in FIG. 1A;
FIG. 5 is an x-ray showing the lateral distal fibula plate shown in FIG. 1 fixed to a broken fibula;
FIG. 6A is a perspective view of a posterolateral distal fibula plate not falling within the scope of the invention;
FIG. 6B is an alternative version of a posterolateral plate not falling within the scope of the invention;
FIG. 7 is a perspective view of a hook plate not falling within the scope of the invention;
FIG. 8 is an enlarged perspective view of a distal end of the hook plate shown in FIG. 7;
FIG. 9 is a side elevational view of the distal end of the hook plate shown in FIG. 8; and
FIGS. 10A-10B depict a universal distal fibula plate not falling within the scope of the invention.

### DETAILED DESCRIPTION

In the drawings, like numerals indicate like elements throughout. Certain terminology is used herein for convenience only and is not to be taken as a limitation on the present invention. The terminology includes the words specifically mentioned, derivatives thereof and words of similar import. The embodiments illustrated below are not intended to be exhaustive or to limit the invention to the precise form disclosed. These embodiments are chosen and described to best explain the principle of the invention and its application and practical use and to enable others skilled in the art to best utilize the invention.

Reference herein to "one embodiment" or "an embodiment" means that a particular feature, structure, or characteristic described in connection with the embodiment can be included in at least one embodiment of the invention. The appearances of the phrase "in one embodiment" in various places in the specification are not necessarily all referring to the same embodiment, nor are separate or alternative embodiments necessarily mutually exclusive of other embodiments. The same applies to the term "implementation."

As used in this application, the word "exemplary" is used herein to mean serving as an example, instance, or illustration. Any aspect or design described herein as "exemplary" is not necessarily to be construed as preferred or advantageous over other aspects or designs. Rather, use of the word exemplary is intended to present concepts in a concrete fashion.

Additionally, the term "or" is intended to mean an inclusive "or" rather than an exclusive "or". That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims should generally be construed to mean "one or more" unless specified otherwise or clear from context to be directed to a singular form.

Unless explicitly stated otherwise, each numerical value and range should be interpreted as being approximate as if the word "about" or "approximately" preceded the value of the value or range.

The use of figure numbers and/or figure reference labels in the claims is intended to identify one or more possible embodiments of the claimed subject matter in order to facilitate the interpretation of the claims. Such use is not to be construed as necessarily limiting the scope of those claims to the embodiments shown in the corresponding figures.

Also for purposes of this description, the terms "couple," "coupling," "coupled," "connect," "connecting," or "connected" refer to any manner known in the art or later developed of joining or connecting two or more elements directly or indirectly to one another, and the interposition of one or more additional elements is contemplated, although not required. Conversely, the terms "directly coupled," "directly connected," etc., imply the absence of such additional elements.

The present disclosure provides embodiments of plates, securing devices, systems that can be used to repair, for example, bone fractures, particularly ankle fractures.

Specifically, embodiments are directed to bone plating with locking and/or non-locking fasteners for dynamic compression of bone. The hole designs may allow for fixed angle and/or polyaxial locking and/or non-locking of the fasteners. Some embodiments include locking fasteners with self-forming threads configured to displace the plate material, thereby locking the fastener to the plate.

While exemplary embodiments of the plates are used to repair ankle fractures, those skilled in the art will recognize that the plates may be adapted to contact one or more of a femur, a distal tibia, a proximal tibia, a proximal humerus, a distal humerus, a clavicle, a fibula, an ulna, a radius, bones of the foot, bones of the hand, or other suitable bone or bones. The bone plate may be curved, contoured, straight, or flat. The plate may have a head portion that is contoured to match a particular bone surface, such as a metaphysis or diaphysis, flares out from the shaft portion, forms an L-shape, T-shape, Y-shape, etc., with the shaft portion, or that forms any other appropriate shape to fit the anatomy of the bone to be treated.

The bone plate may be comprised of titanium, stainless steel, cobalt chrome, carbon composite, plastic or polymer-such as polyetheretherketone (PEEK), polyethylene, ultra high molecular weight polyethylene (UHMWPE), resorbable polylactic acid (PLA), polyglycolic acid (PGA), combinations or alloys of such materials or any other appropriate material that has sufficient strength to be secured to and hold bone, while also having sufficient biocompatibility to be implanted into a body. Similarly, the fasteners may be comprised of titanium, cobalt chrome, cobalt-chrome-molybdenum, stainless steel, tungsten carbide, combinations or alloys of such materials or other appropriate biocompatible materials. Although the above list of materials includes many typical materials out of which bone plates and fasteners are made, it should be understood that bone plates and fasteners comprised of any appropriate material are contemplated.

The embodiments of the disclosure and the various features and advantageous details thereof are explained more fully with reference to the nonlimiting embodiments and examples that are described and/or illustrated in the accompanying drawings and detailed in the following description. The features of one embodiment may be employed with other embodiments as the skilled artisan would recognize, even if not explicitly stated herein. Descriptions of well-known components and processing techniques may be omitted so as to not unnecessarily obscure the embodiments of the disclosure. The examples used herein are intended merely to facilitate an understanding of ways in which the disclosure may be practiced and to further enable those of skill in the art to practice the embodiments of the disclosure. Accordingly, the examples and embodiments herein should not be construed as limiting the scope of the disclosure, which is defined solely by the appended claims and applicable law.

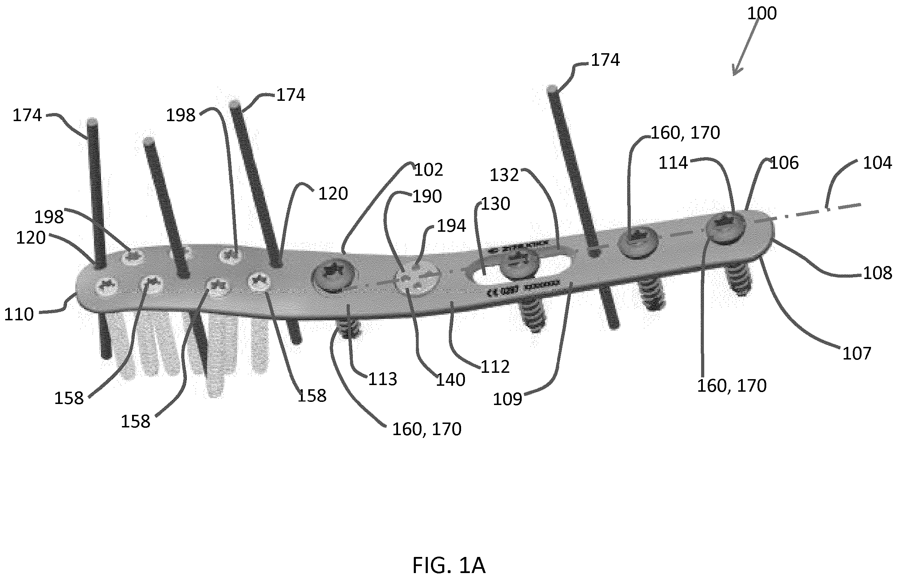

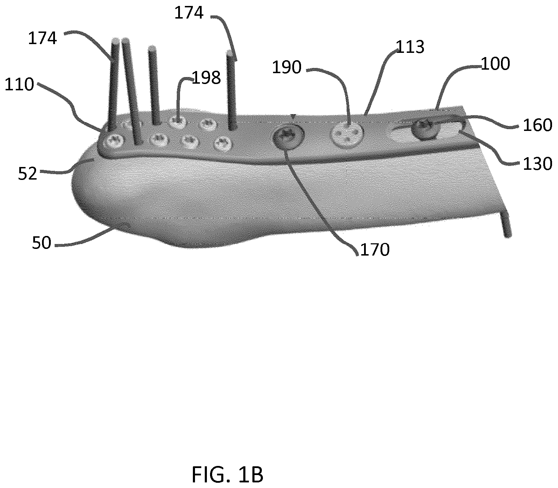

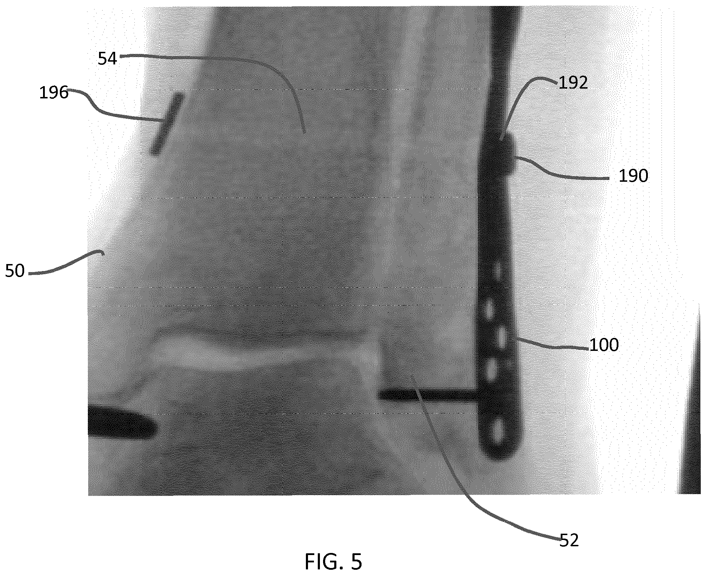

Referring to FIGS. 1-5, a lateral distal fibula plate 100 ("plate 100") according to a first exemplary embodiment is shown. Referring specifically to FIGS. 1B and 5, plate 100 is fixed to the lateral surface 52 of a fibula 50. Plate 100 may be used to treat fractures of the distal fibula 50 and/or disruption of the syndesmosis, and can have a pre-contoured shape, such as is shown in FIG. 1A. Alternatively, plate 100 can be contoured prior to use in order to conform to a particular bone structure.

Plate 100 has an elongate body 102 extending generally along a central longitudinal axis 104. Plate 100 has an upper surface 106 extending between a proximal end 108 and a distal end 110 and a lower surface 107 configured to be in contact with bone. A body portion 112 extends between proximal end 108 and distal end 110 and has a transition section 113 where body portion 112 transitions from a generally planar portion 109 proximate to proximal end 108 and a contoured portion 111 proximate to distal end 110.

As used herein, the term "contoured" means "curved" such that contoured portion 111 includes surfaces (upper surface 106, lower surface 107, or both) with non-infinite radii. The contours do not necessarily need to be constant; the radius of curvature of contoured portion 111 can vary along the length and width of contoured portion 111. In an exemplary embodiment, contoured portion 111 can be contoured to match the contours of the bone to which plate 100 is to be fixed, such as a fibula.

In an exemplary embodiment, proximal end 108 and distal end 110 each includes a smooth, rounded ends and edges. Body portion 112 is contoured, with smooth, rounded edges. The smooth, rounded ends and edges eliminate the potential for inadvertently engaging and ripping any adjoining tissue.

Body portion 112 also includes a plurality of different types of through-openings formed therein and extending from upper surface 106 to lower surface 107. The different types of through-openings disclosed in plate 100 will discussed from proximal end 108 to distal end 110, although those skilled in the art will recognize that the through-openings can be located at different places, in different orders, and intermixed together throughout the length of plate 100.

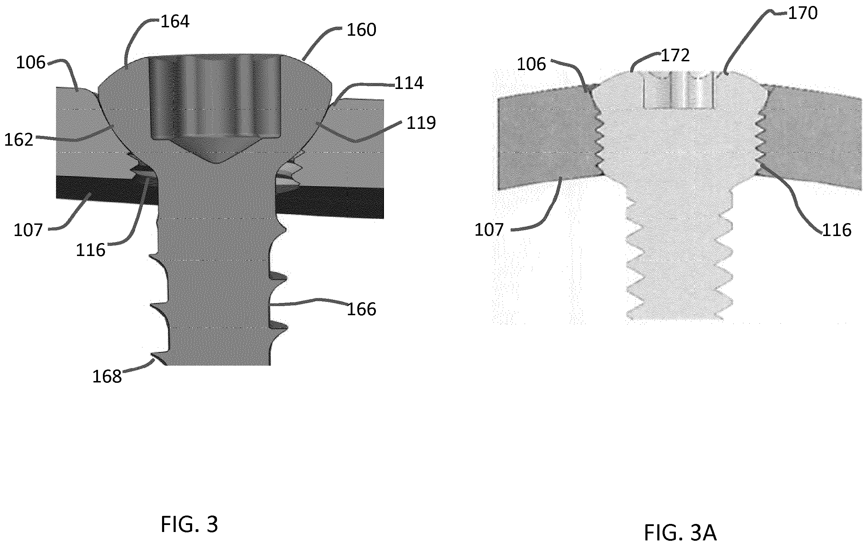

Referring to FIG. 3, through-openings 114 are shaft holes including a threaded portion 116 proximate to lower surface 107 and a non-threaded portion 118 proximate to upper surface 106. Through-openings 114 may extend along longitudinal axis 104.

Threaded portion 116 and non-threaded portion 118 are co-axial. The shaft holes can accept both locking and non-locking screws, resulting in a "stacked" design, in which a non-locking hole geometry, non-threaded portion 118, is on top of locking threaded portion 116 below.

Through-openings 114 can alternatively receive fasteners comprised of locking screws or non-locking (compression) screws. In exemplary embodiments, screw 160 can be 3.5 mm or 4.0 mm screws, for example.

FIG. 3 shows a non-locking screw 160 inserted into through-opening 114. Non-threaded portion 118 is generally conical in shape such that non-threaded portion 118 is wider near upper surface 106 of plate 100 and narrower toward threaded portion 116. Screw 160 has a substantially smooth convex portion 162 of a head 164 configured to be received by and engage with non-threaded portion 118 and to dynamically compress bone 50 after fixation of plate 100 to bone 50. Non-threaded portion 118 has a generally concave surface 119 to mate with convex surface portion 162 of head 164 of screw 160.

A shaft 166 of screw 160 has distal threads 168 that are configured to screw into bone 50. Shaft 166 and threads 168 have a narrower diameter than that of through-opening 114 so that shaft 166 can pass through through-opening 114 without engaging threaded portion 116 of through-opening 114.

A locking screw 170 is shown in FIG. 3A. Locking screw 170 has a threaded head portion 172 configured to engage threaded portion 116 of through-opening 114 and to lock screw 170 to plate 100. In some embodiments, threaded head portion 172 is a self-forming thread that is configured to displace material in threaded portion 116 of plate 100 to lock fastener 170 to plate 100.

A second type of through-opening 120, shown in FIG. 1A, may be sized and dimensioned to allow a K-wire 174 to pass therethrough. In an exemplary embodiment, through-opening 120 is sized to allow a 1.6 mm K-wire, to pass therethrough, although those skilled in the art will recognize that other size through-openings 120 can be provided for other size K-wires. As noted in FIG. 1A, one or more through-openings 120 are spaced along a length of plate 100 and are not necessarily aligned with longitudinal axis 104.

A third type of through-opening that can be provided in plate 100 is an elongate slot 130. Elongate slot 130 may extend along longitudinal axis 104, for example. The elongated slot 130 may have a length greater than its width, for example, a length two times its width, a length three times its width, or more. Elongate slot 130 may allow for a range of securing member insertion locations. In an exemplary embodiment, one elongate through-opening 130 is provided, although those skilled in the art will recognize, depending on the length of plate 100 and through-opening 130, one or more than through-opening 130 can be provided.

Slots 130 include generally smooth side walls to allow a securing member, such as screw 160, to be inserted at infinite locations along the length of each slot 130. A rib 132 extends around the inner perimeter of slot 130 below upper surface 106. In an exemplary embodiment, screws 130 can be 3.5 mm or 4.0 mm non-locking screws and can provide up to 1 mm of compression or distraction. Screws 160 may engage rib 132 along under surface 162 of head 164 of screw 160 so that head 164 is largely, if not entirely, within slot 130 to minimize the amount of head 162 extending above upper surface 106 of plate 100.

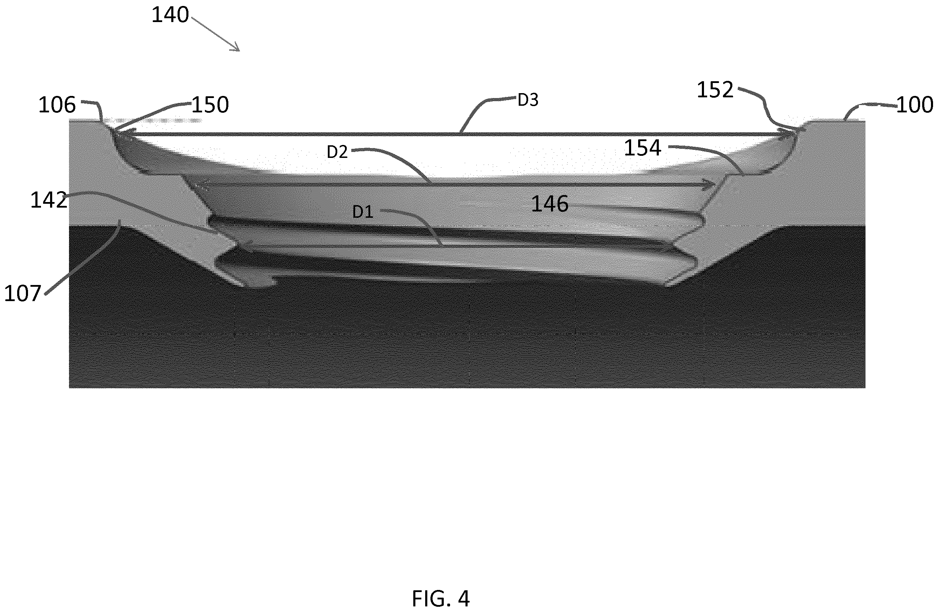

Referring to FIGS. 1A and 4, a fourth type of through-opening that can be provided in plate 100 is a syndesmotic hole 140 located at transition portion 113. Syndesmotic hole 140 can accept three different types of fixation: (1) a suture button 190; (2) non-locking screw 160; or (3) locking screw 170.

Referring specifically to the cross-section of hole 140 in FIG. 4, hole 140 includes, from lower surface 107 of plate 100 to upper surface 106 of plate 100, at least three different co-axial bores. A first bore includes a threaded portion 142, similar to threaded portion 116 of through-opening 114, with its bore having a first diameter D1.

Similar to through-opening 114, a second bore of hole 140 is has an unthreaded conical portion 144 with a conical side wall 146 located above and adjacent to threaded portion 142. The second bore of hole 140 has a maximum diameter D2, larger than diameter D1.

In contrast to through-opening 114, hole 140 further includes a third bore comprising a bowl portion 150 having a diameter D3, larger than maximum diameter D2. Bowl portion 150 is for the use of a suture button system that includes, for example, two metal buttons 190, 196, connected via suture. Button 196 interfaces with the far cortex of bone 50 (shown in FIG. 5), while button 190 interfaces with bone plate 100. This button system provides stability for a disrupted joint or bone fracture and also provides a type of mobile stability. The use of buttons 190, 196 may be a method of treatment for the syndesmosis when the syndesmosis is disrupted. Another type of fixation of a disrupted syndesmosis is a bone screw as described above, which is a more rigid fixation than the suture button system.

Referring to FIG. 4, bowl portion 150 is above and adjacent to conical portion 146. Conical portion 146 has a maximum diameter of diameter D2 proximate to bowl portion 150. Bowl portion 150 comprises a bowl diameter, larger than the maximum diameter. The transition between bowl portion 150 and threaded portion 142 (i.e. conical portion 144) is shown in FIG. 4 as a chamfer, but could alternatively be a round.

Bowl portion 150 includes a side wall 152 that circumscribes bowl portion 150 and an annular surface 154 between the side wall 152 and conical portion 144. Annular surface 154 surrounds side wall 146 of conical portion 144. Bowl portion 150 is configured to receive and retain a button 190 having a lower surface 192 (shown in FIG. 5) configured to engage annular surface 154 and fit within side wall 152.

Referring to FIG. 1A, button 190 also includes at least one thread hole 194 that is in communication with hole 140 when lower surface 192 is in engagement with annular surface 154.

Referring to FIG. 5, first button 190 is configured for insertion into bowl portion 150 of hole 140 and a second button 196 is configured to engage bone 50, distal from bone plate 100. A suture (not shown) extends through thread hole 194, through plate 100 and a passage 54 drilled through bone 50, to second button 196 to compress bone 50 and plate 100 between buttons 190, 196.

A fifth set of through holes 158 are provided at distal end 110 of plate 100. Holes 158 may be configured to receive locking screws 198. In an exemplary embodiment, holes 158 may be threaded to accept 2.5mm locking screws 198, for example. A plurality of holes 158 (about seven as shown in plate 100) are provided to fix distal end 110 of plate 100 securely in bone 50. Holes 158 are not constrained along longitudinal axis 104 but instead are located along the width of plate 100 to provide a plurality of screw connections for a secure fixation to bone.

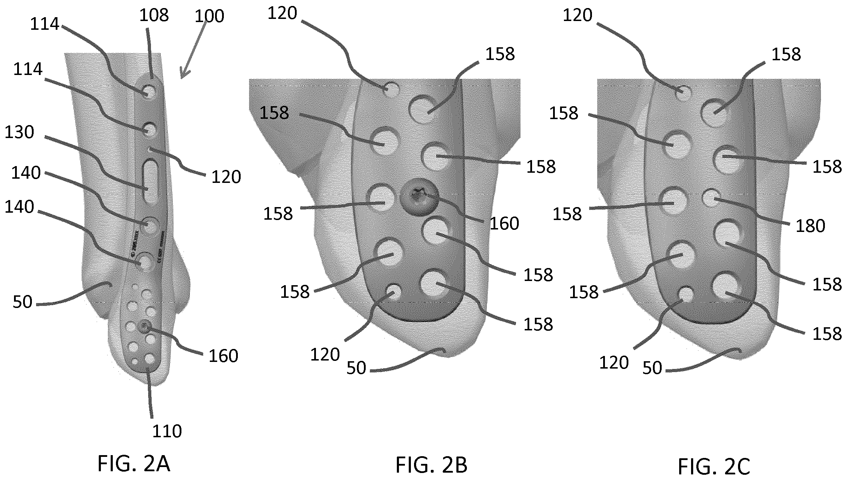

FIGS. 2A depicts an alternative version of a lateral distal fibula plate 100. FIGS. 2B and 2C show close up views of the distal portion of the plate 100. The plate 100 is substantially the same as that disclosed in FIG. 1A, with the addition of a sixth type of through hole 180. This through hole 180 may be a non-threaded hole located within the distal head of the plate 100. Through hole 180 may have smaller diameter than the other distal through holes 158. The through hole 180 may be positioned near or surrounded by distal locking holes 158. The through hole 180 may be configured accept a non-locking fastener or screw 160 that can act as a suck-down screw. In a preferred embodiment, this hole 180 is not threaded. When used, the non-locking screw 160 may be placed first in order to get the plate 100 to be compressed down to the bone 50. This screw 160 can be left in after the other distal locking screws 198 are placed in holes 158 or screw 160 can be removed. As shown in FIGS. 2A-2C, the middle non-threaded hole 180 is configured to accept the non-locking screw 160, but it is envisioned that either a proximal or distal non-threaded hole 180 could accept a non-locking screw 160.

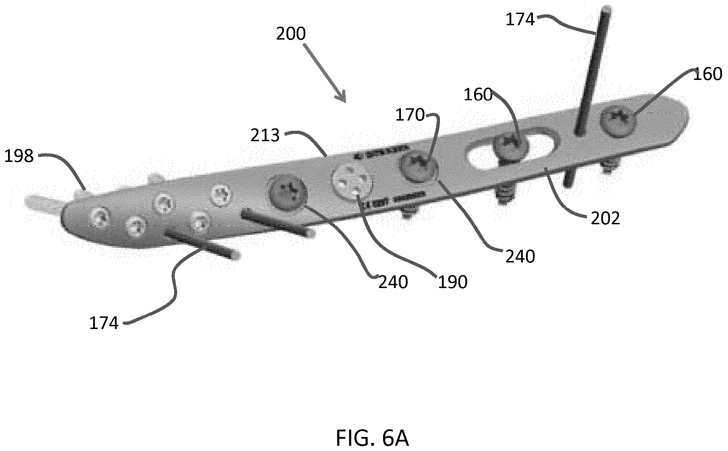

A second embodiment of a plate 200, shown in FIG. 6A, is a posterolateral distal fibula plate. Plate 200 sits on the posterior face of the fibula distally, and wraps around to the lateral surface as plate 200 travels proximally. Plate 200 can be used to facilitate a posterior surgical approach. Similar to plate 100, plate 200 can have a variety of different through-openings, including syndesmotic holes 240, similar to syndesmotic holes 140 described above.

Plate 200 has a generally planar body 202 with a transition portion 213 proximate to syndesmotic holes 240, where body 202 transitions to a contoured shape to conform to the posterior face of the fibula.

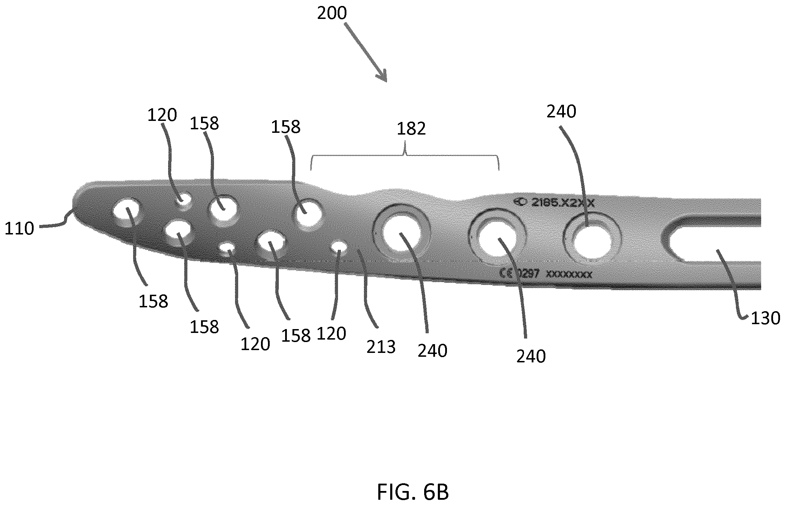

FIG. 6B depicts an alternative version of posterolateral distal fibula plate 200. The plate 200 is substantially the same as that disclosed in FIG. 6A, with the addition of one or more scalloped edges 182. The scalloped edge or edges 182 may be positioned along the transition region 213 of the plate 200. The scalloped edge 182 may extend along one or both sides of the plate 200. As shown, the scalloped edge 182 may be positioned on a first side of the plate 200. The scalloped edge 182 may include a first recess or cutout positioned along the edge between the second and third syndesmotic holes 240 and a second recess or cutout positioned along the edge between the third syndesmotic hole 240 and the first locking hole 158. Because the posterolateral plate 200 is configured to sit on the posterior side of the fibula 50, syndesmotic screws 160, 170 (placed lateral to medial) or suture buttons 190 often cannot be placed through the plate 200. In some embodiments, these scallops 182 are configured to allow additional screws or suture buttons to be placed outside of the plate 200 and aids in these treatment options by not directly contacting the plate 200. In some embodiments, one or more fasteners (e.g., screws 160, 170) may be placed outside the plate 200, but in close proximity or in contact with the scalloped edge 182.

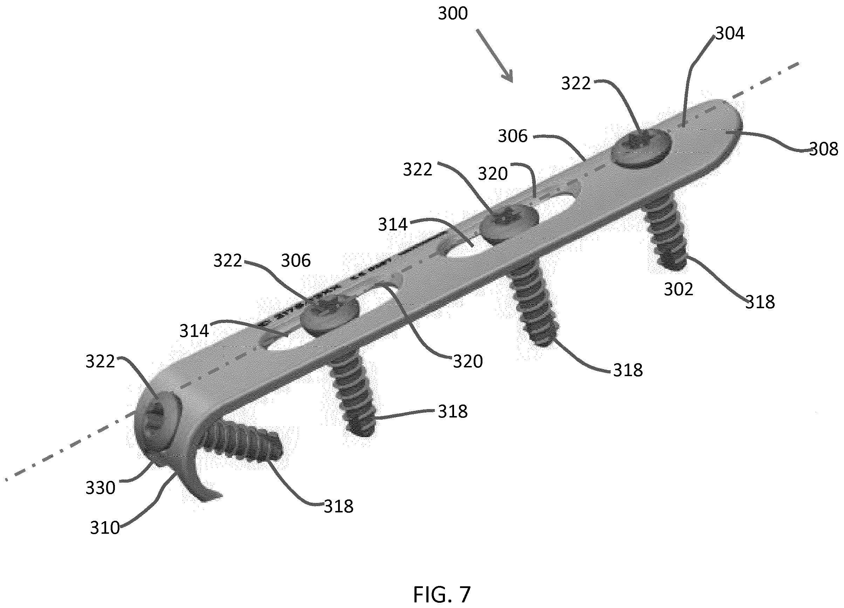

A third embodiment of a hook plate 300 ("plate 300") is shown in FIGS. 7-9. Plate 300 can be used for very distal fractures of the tibia or fibula, for example. Hook plate 300 has an elongate body 302 extending generally along a central longitudinal axis 304. Plate 300 has a generally planar top surface 306 extending between a proximal end 308 and a distal end 310. A body portion 312 extends between proximal end 308 and distal end 310. Plate 300 is symmetrical about a plane extending through central longitudinal axis 304 perpendicular to top surface 306.

In an exemplary embodiment, proximal end 308 includes a smooth, rounded face. The smooth, rounded face eliminates the potential for inadvertently engaging and ripping any adjoining tissue.

Body portion 312 is generally planar, with smooth, rounded surfaces, again to eliminate the potential for inadvertently engaging and ripping any adjoining tissue. Body portion 312 also includes a plurality of through-openings 314 formed therein. Through-openings 314 are elongate slots and allow for a range of securing member insertion locations. In an exemplary embodiment, two elongate through-openings 314 are provided, although those skilled in the art will recognize, depending on the length of plate 300 and through-openings 314, more or less than two through-openings 314 can be provided.

Through-openings 314 include generally smooth side walls to allow securing members 318 to be inserted at infinite locations along the length of each through-opening 314. A rib 320 may extend around the inner perimeter of through-opening 314 below top surface 306. In an exemplary embodiment, securing members 318 can be 3.5 mm or 4.0 mm non-locking screws and can provide up to 1 mm of compression or distraction. Securing members 318 engage rib 320 along an under surface of the head 322 of securing member 318 so that head 322 is largely, if not entirely, within through-opening 314 to minimize the amount of head 322 extending above top surface 306 of plate 300.

Through-openings 316 may be located at either end of plate 300. Through-openings 316 are shaft holes that can accept either one of locking and non-locking screws via the "stacked" design described above. A first through-opening 316 is located at proximal end 308 and a second through-opening 316 is located at distal end 310.

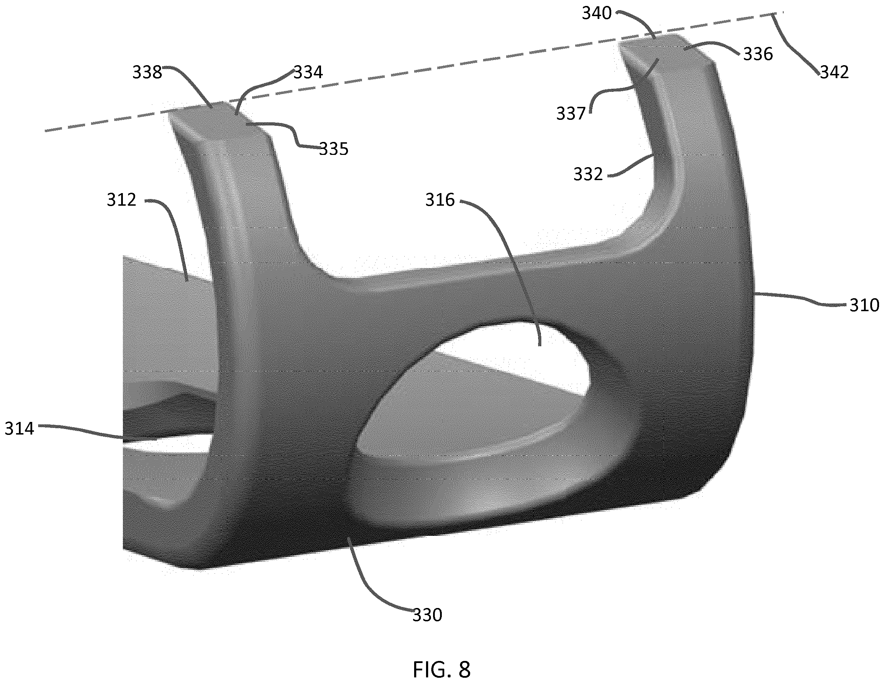

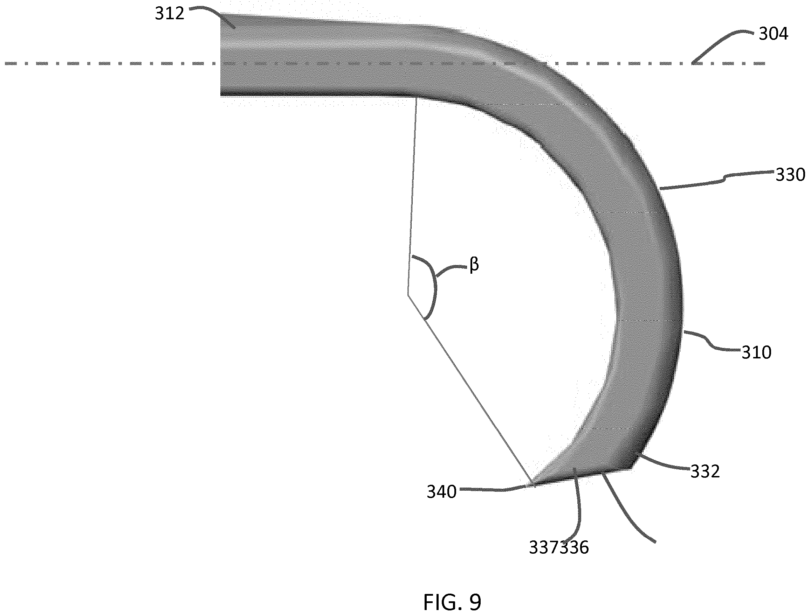

Referring to FIGS. 8 and 9, distal end 310 includes an arcuate surface 330 that extends away from the plane of body portion 312, and is used to capture the distal bone fragment of either the tibia or the fibula. Arcuate surface 330 extends in an arc having an angle β of between about 100 degrees and about 160 degrees, about 125 degrees and about 155 degrees, or about 135 degrees and about 150 degrees from body portion 312. At least one through-opening 316 extends through arcuate surface 330.

A most distal end 332 of arcuate surface 330 includes a hook assembly having two separate hooks 334, 336. Each hook 334, 336 includes a flat surface 335, 337, respectively and each flat surface 335, 337 includes a corresponding cutting edge 338, 340, respectively. Cutting edges 338, 340 extend along a single line 342 that is perpendicularly skew to longitudinal axis 304 and are used to engage and dig into bone material in the tibia or fibula.

With the exception of cutting edges 338, 340, all edges of arcuate surface 330 and hooks 334, 336 have smooth, rounded surfaces, again to eliminate the potential for inadvertently engaging and ripping any adjoining tissue.

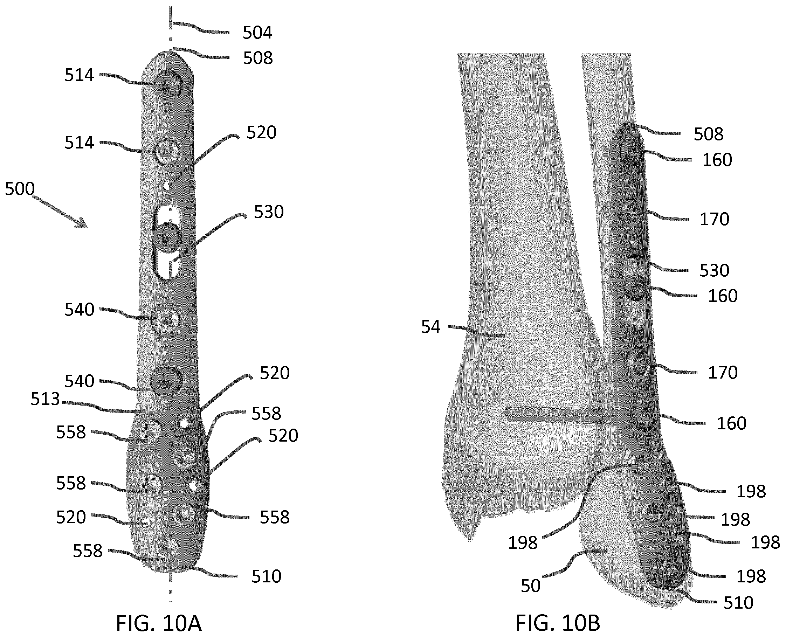

FIGS. 10A depicts an embodiment of a universal distal fibula plate 500. In FIG. 10B, the universal distal fibula plate 500 is positioned against a distal portion of the fibula 50. This plate 500 is similar to the lateral distal fibula plate 100 described above. Plate 500 has an elongate body 502 extending generally along a central longitudinal axis 504. The plate 500 has a transition section 513 between proximal end 508 and distal end 510 where body 502 transitions from a generally planar portion proximate to proximal end 508 to an enlarged head portion proximate to distal end 510. The enlarged head portion may have a width greater than the width of the remainder of the plate 500. The enlarged distal head portion of the plate 500 may be bent or contoured to mimic the anatomy of the fibula 50 (e.g. the lateral malleolus of the fibula). In an exemplary embodiment, proximal end 508 and distal end 510 each includes smooth, rounded ends and edges. The plate 500 is generally symmetric about the longitudinal axis 504, such that plate 500 can accommodate left or right fibulas.

Similar to plate 100, plate 500 includes a plurality of different types of through-openings formed therein and extending therethrough. Through-openings 514, similar to through-openings 114 described herein, may feature a "stacked" design where the holes can accept both locking and non-locking fasteners 160, 170 or screws. In exemplary embodiments, the openings 114 are configured to accept fasteners including 3.5 mm or 4.0 mm screws, for example. Through-openings 514 may extend along longitudinal axis 504. Through-openings 520, similar to through-openings 120, may be sized and dimensioned to allow a K-wire to pass therethrough. Through-opening 530, similar to elongate slot 130, may extend along longitudinal axis 504, for example. Syndesmotic openings 540, similar to syndesmotic holes 140, are each configured to accept a suture button 190, a non-locking screw 160, or a locking screw 170. In particular, the syndesmotic holes 540 can accept 3.5mm or 4.0mm locking or non-locking screws. As best seen in FIG. 10B, at least one of the fasteners is positioned through the syndesmotic hole 540 has a length sufficient to engage the fibula 50 and the tibia 54. As shown, a non-locking fastener 160 connects the plate 500 to the fibula 50 and the tibia 54. In this embodiment, the other fasteners 160, 170 have a length sufficient to only engage the fibula 50.

Through-openings 558, similar to through holes 158, are provided in the enlarged distal head portion at the distal end 510 of plate 500. These holes 558 may be conically-threaded locking holes configured to receive locking fasteners 198. Holes 558 may be located along the width of plate 500 to provide a plurality of screw connections for a secure fixation to the fibula 50. One or more of the holes 558 may be replaced with a non-threaded hole 180 as described for the lateral distal fibula plate 100, which may be configured accept a non-locking fastener or screw 160 that can act as a suck-down screw. Plate 500 is universal and configured to secure the bone on left or right fibulas.

The bone plates 100, 200, 300, 500 described herein may be especially configured for treatment of an ankle fracture. In particular, these plates 100, 200, 300, 500 may be especially suitable for treatment of the distal fibula including lateral distal fibula or the posterolateral distal fibula, and/or the distal tibia. These anatomic bone plates 100, 200, 300, 500 may facilitate improved treatment methods of ankle fractures and can provide a number of treatment options based on surgeon preference.

Although the invention has been described in detail and with reference to specific embodiments, it will be apparent to one skilled in the art that various changes and modifications can be made without departing from the scope of the invention. Thus, it is intended that the invention covers the modifications and variations of this invention provided they come within the scope of the appended claims. Thus, the features of one embodiment may be added or combined with the features of another embodiment.

## Claims

1. A stabilization system comprising:
a bone plate (100; 500) having an elongate body (102; 502) extending from a proximal end (108; 508) to a distal end (110; 510) along a longitudinal axis (104; 504) and having an enlarged head portion proximate the distal end (110; 510), wherein the bone plate is generally symmetric about the longitudinal axis (104; 504),
wherein the bone plate (100; 500) having an elongated slot (130; 530) located along the longitudinal axis having a length greater than its width,
wherein the bone plate (100; 500) has a plurality of syndesmotic openings (140; 540) located along the longitudinal axis (104; 504), wherein one of the syndesmotic openings (140; 540) is located adjacent to the elongated slot (130; 530), the syndesmotic openings (140; 540) being sized and dimensioned to accept a suture button (190), a non-locking screw (160), or a locking screw (170),
wherein each of the syndesmotic openings (140; 540) includes from a lower surface (107) of the plate (100) to upper surface (106) of the plate (100) at least three different co-axial bores,
wherein the bone plate having a plurality of openings (120; 520) in the enlarged head portion, **characterized in that**
the at least three co-axial bores_include a first bore including a threaded portion (142) having a first diameter (D1), a second bore having an unthreaded conical portion (144) with conical side wall (146) located above and adjacent to the threaded portion (142) having a maximum diameter (D2) larger than diameter (D1), and a third bore comprising a bowl portion (150) above and adjacent to conical portion (146) having a diameter (D3) larger than the maximum diameter (D2) proximate to the bowl portion (150), the bowl portion (150) includes a side wall (152) that circumscribes bowl portion (150) and an annular surface (154) between the side wall (152) and conical portion (144), the annular surface (154) surrounding a side wall (146) of the conical portion (144), wherein the bowl portion (150) is configured to receive and retain a button (190) having a lower surface (192) configured to engage the annular surface (154) and fit within side wall (152).

2. The stabilization system according to claim 1 wherein the body portion extends between the proximal end (108) and the distal end (110) and has a transition section (113) where the body portion transitions from a generally planar portion (109) proximate to the proximal end (108) and a contoured portion (111) proximate to distal end (110), wherein the syndesmotic opening (140) is located at the transition portion (113).

3. The stabilization system according to claim 1, further comprising a first fastener (160) configured to engage the elongated slot (130; 530) to secure the bone plate (100; 500) to the bone.

4. The stabilization system according to claim 1, wherein the plurality of openings (520) in the enlarged head portion comprise conically-threaded locking holes (558).

5. The stabilization system according to claim 1, wherein one of the plurality of openings (520) in the enlarged head portion is a non-threaded hole configured to receive a non-locking fastener in order to compress the plate (100; 500) to the bone.

6. The stabilization system according to claim 1, wherein an edge of the bone plate (100; 500) includes a scalloped section.

7. The stabilization system according to claim 6, wherein the plurality of syndesmotic openings (140; 540) includes first, second, and third syndesmotic openings, wherein the plurality of openings (520) in the enlarged head portion includes a first locking hole positioned proximate to the third syndesmotic opening, and wherein the scalloped section includes a first cutout positioned along the edge between the second and third syndesmotic holes and a second cutout positioned along the edge between the third syndesmotic hole and the first locking hole.

8. The stabilization system according to claim 1, wherein the bone plate (100; 500) is configured to contact the distal fibula.

## Patentansprüche

1. Stabilisierungssystem, umfassend:
- eine Knochenplatte (100; 500) mit einem länglichen Körper (102; 502), der sich von einem proximalen Ende (108; 508) zu einem distalen Ende (110; 510) entlang einer Längsachse (104; 504) erstreckt und einen vergrößerten Kopfabschnitt in der Nähe des distalen Endes (110; 510) aufweist, wobei die Knochenplatte im Wesentlichen symmetrisch um die Längsachse (104; 504) ist,
- wobei die Knochenplatte (100; 500) einen länglichen Schlitz (130; 530) aufweist, der entlang der Längsachse angeordnet ist und eine Länge aufweist, die größer ist als seine Breite,
- wobei die Knochenplatte (100; 500) eine Mehrzahl von Syndesmoseöffnungen (140; 540) aufweist, die entlang der Längsachse (104; 504) angeordnet sind, wobei eine der Syndesmoseöffnungen (140; 540) neben dem Längsschlitz (130; 530) angeordnet ist, wobei die Syndesmoseöffnungen (140; 540) bemessen und dimensioniert sind, um einen Wundnahtknopf (190), eine nicht verriegelnde Schraube (160) oder eine verriegelnde Schraube (170) aufzunehmen,
- wobei jede der Syndesmoseöffnungen (140; 540) von einer unteren Fläche (107) der Platte (100) bis zu einer oberen Fläche (106) der Platte (100) zumindest drei verschiedene koaxiale Bohrungen umfasst,
- wobei die Knochenplatte eine Mehrzahl von Öffnungen (120; 520) im vergrößerten Kopfabschnitt aufweist, **dadurch gekennzeichnet, dass**
- die zumindest drei koaxialen Bohrungen eine erste Bohrung mit einem Gewindeabschnitt (142) mit einem ersten Durchmesser (D1), eine zweite Bohrung mit einem gewindelosen konischen Abschnitt (144) mit einer konischen Seitenwand (146), die oberhalb und neben dem Gewindeabschnitt (142) angeordnet ist und einen maximalen Durchmesser (D2) aufweist, der größer ist als der Durchmesser (D1), und eine dritte Bohrung mit einem schalenförmigen Abschnitt (150) oberhalb und neben dem konischen Abschnitt (146) umfassen, der einen Durchmesser (D3) aufweist, der größer ist als der maximale Durchmesser (D2) in der Nähe des schalenförmigen Abschnitts (150), wobei der schalenförmige Abschnitt (150) eine Seitenwand (152), die den schalenförmigen Abschnitt (150) umgibt, und eine ringförmige Fläche (154) zwischen der Seitenwand (152) und dem konischen Abschnitt (144) umfasst, wobei die ringförmige Fläche (154) eine Seitenwand (146) des konischen Abschnitts (144) umgibt, wobei der schalenförmige Abschnitt (150) eingerichtet ist, um einen Knopf (190) aufzunehmen und zu halten, der eine untere Fläche (192) aufweist, die eingerichtet ist, um mit der ringförmigen Fläche (154) in Eingriff zu stehen und in die Seitenwand (152) zu passen.

2. Stabilisierungssystem nach Anspruch 1, wobei sich der Körperabschnitt zwischen dem proximalen Ende (108) und dem distalen Ende (110) erstreckt und einen Übergangsbereich (113) aufweist, wo der Körperabschnitt von einem im Wesentlichen ebenen Abschnitt (109) in der Nähe des proximalen Endes (108) in einen konturierten Abschnitt (111) in der Nähe des distalen Endes (110) übergeht, wobei die Syndesmoseöffnung (140) am Übergangsbereich (113) angeordnet ist.

3. Stabilisierungssystem nach Anspruch 1, das ferner ein erstes Befestigungselement (160) aufweist, das eingerichtet ist, um mit dem Längsschlitz (130; 530) in Eingriff zu stehen, um die Knochenplatte (100; 500) am Knochen zu befestigen.

4. Stabilisierungssystem nach Anspruch 1, wobei die Mehrzahl von Öffnungen (520) im vergrößerten Kopfabschnitt Verriegelungslöcher (558) mit konischem Gewinde aufweist.

5. Stabilisierungssystem nach Anspruch 1, wobei eine der Mehrzahl von Öffnungen (520) im vergrößerten Kopfabschnitt ein gewindeloses Loch ist, das eingerichtet, um ein nicht verriegelndes Befestigungselement aufzunehmen, um die Platte (100; 500) an den Knochen zu drücken.

6. Stabilisierungssystem nach einem der Ansprüche 1 bis 5, wobei ein Rand der Knochenplatte (100; 500) einen bogenförmigen Bereich aufweist.

7. Stabilisierungssystem nach Anspruch 6, wobei die Mehrzahl von SyndesmoseÖffnungen (140; 540) eine erste, eine zweite und eine dritte Syndesmoseöffnung umfasst, wobei die Mehrzahl von Öffnungen (520) im vergrößerten Kopfabschnitt ein erstes Verriegelungsloch umfasst, das in der Nähe der dritten Syndesmoseöffnung positioniert ist, und wobei der bogenförmige Bereich einen ersten Ausschnitt umfasst, der entlang der Kante zwischen den zweiten und dritten Syndesmoseöffnungen angeordnet ist, und einen zweiten Ausschnitt umfasst, der entlang der Kante zwischen der dritten Syndesmoseöffnung und dem ersten Verriegelungsloch angeordnet ist.

8. Stabilisierungssystem nach Anspruch 1, wobei die Knochenplatte (100; 500) eingerichtet ist, um die distale Fibula zu berühren.

## Revendications

1. Système de stabilisation comprenant
une plaque osseuse (100 ; 500) ayant un corps allongé (102 ; 502) s'étendant d'une extrémité proximale (108 ; 508) à une extrémité distale (110 ; 510) le long d'un axe longitudinal (104 ; 504) et ayant une partie de tête élargie à proximité de l'extrémité distale (110 ; 510), dans laquelle la plaque osseuse est généralement symétrique par rapport à l'axe longitudinal (104 ; 504),
la plaque osseuse (100 ; 500) ayant une fente allongée (130 ; 530) située le long de l'axe longitudinal et dont la longueur est supérieure à la largeur,
dans lequel la plaque osseuse (100 ; 500) a une pluralité d'ouvertures syndesmotiques (140 ; 540) situées le long de l'axe longitudinal (104 ; 504), l'une des ouvertures syndesmotiques (140 ; 540) étant située à côté de la fente allongée (130 ; 530), les ouvertures syndesmotiques (140 ; 540) étant dimensionnées pour recevoir un bouton de suture (190), une vis non verrouillable (160), ou une vis verrouillable (170),
dans lequel chacune des ouvertures syndesmotiques (140 ; 540) inclut, depuis la surface inférieure (107) de la plaque (100) jusqu'à la surface supérieure (106) de la plaque (100), au moins trois alésages coaxiaux différents,
dans lequel la plaque osseuse ayant une pluralité d'ouvertures (120 ; 520) dans la partie élargie de la tête, **caractérisée par le fait que**
les au moins trois alésages coaxiaux incluent un premier alésage incluant une partie filetée (142) ayant un premier diamètre (D1), un deuxième alésage ayant une partie conique non filetée (144) avec une paroi latérale conique (146) située au-dessus et adjacente à la partie filetée (142) ayant un diamètre maximal (D2) supérieur au diamètre (D1), et un troisième alésage comprenant une partie de cuvette (150) au-dessus et adjacente à la partie conique (146) ayant un diamètre (D3) supérieur au diamètre maximal (D2) à proximité de la partie de cuvette (150), la partie bol (150) inclut une paroi latérale (152) qui circonscrit la partie bol (150) et une surface annulaire (154) entre la paroi latérale (152) et la partie conique (144), la surface annulaire (154) entourant une paroi latérale (146) de la partie conique (144), dans laquelle la partie bol (150) est configurée pour recevoir et retenir un bouton (190) ayant une surface inférieure (192) configurée pour s'engager dans la surface annulaire (154) et s'adapter à l'intérieur de la paroi latérale (152).

2. Système de stabilisation selon la revendication 1 dans lequel la partie du corps s'étend entre l'extrémité proximale (108) et l'extrémité distale (110) et a une section de transition (113) où la partie du corps passe d'une partie généralement plane (109) proche de l'extrémité proximale (108) à une partie profilée (111) proche de l'extrémité distale (110), dans laquelle l'ouverture syndesmotique (140) est située au niveau de la partie de transition (113).

3. Système de stabilisation selon la revendication 1, comprenant en outre une première fixation (160) configurée pour s'engager dans la fente allongée (130 ; 530) afin de fixer la plaque osseuse (100 ; 500) à l'os.

4. Système de stabilisation selon la revendication 1, dans lequel la pluralité d'ouvertures (520) dans la partie élargie de la tête comprend des trous verrouillables à filetage conique (558).

5. Système de stabilisation selon la revendication 1, dans lequel l'une des ouvertures (520) de la partie élargie de la tête est un trou non fileté configuré pour recevoir une attache non verrouillable afin de comprimer la plaque (100 ; 500) sur l'os.

6. Système de stabilisation selon la revendication 1, dans lequel un bord de la plaque osseuse (100 ; 500) inclut une section festonnée.

7. Système de stabilisation selon la revendication 6, dans lequel la pluralité d'ouvertures syndesmotiques (140 ; 540) inclut des première, deuxième et troisième ouvertures syndesmotiques, dans lequel la pluralité d'ouvertures (520) dans la partie élargie de la tête inclut un premier trou verrouillable positionné à proximité de la troisième ouverture syndesmotique, et dans lequel la section festonnée inclut une première découpe positionnée le long du bord entre les deuxième et troisième trous syndesmotiques et une deuxième découpe positionnée le long du bord entre le troisième trou syndesmotique et le premier trou verrouillable.

8. Système de stabilisation selon la revendication 1, dans lequel la plaque osseuse (100 ; 500) est configurée pour entrer en contact avec le péroné distal.
